(19) Europäisches Patentamt

**European Patent Office**

Office européen des brevets

(11) Publication number: **0 181 374 B1**

# (12) EUROPEAN PATENT SPECIFICATION

(45) Date of publication of patent specification: **21.11.91**    (51) Int. Cl.⁵: **C12Q 1/70**

(21) Application number: **85902364.0**

(22) Date of filing: **23.04.85**

(86) International application number:
**PCT/US85/00762**

(87) International publication number:
**WO 85/04903 (07.11.85 85/24)**

The file contains technical information submitted after the application was filed and not included in this specification

(54) **ISOLATION OF PROTEINS OF HTLV-III, SEROLOGICAL DETECTION OF ANTIBODIES TO HTLV-III IN SERA OF PATIENTS WITH AIDS AND PRE-AIDS CONDITIONS, AND DETECTION OF HTLV-III INFECTION BY IMMUNO-ASSAYS USING HTLV-III AND ITS PROTEINS.**

(30) Priority: **23.04.84 US 602945**
         **30.07.84 US 635610**
         **24.08.84 US 643715**

(43) Date of publication of application:
**21.05.86 Bulletin 86/21**

(45) Publication of the grant of the patent:
**21.11.91 Bulletin 91/47**

(84) Designated Contracting States:
**AT BE CH DE FR GB IT LI LU NL SE**

(56) References cited:
**EP-A- 0 116 949**
**EP-A- 0 136 798**
**EP-A- 0 138 667**

**JNCI, vol. 69, no. 4, October 1982, pp. 981-984**

(73) Proprietor: **THE UNITED STATES OF AMERICA represented by The Secretary The United States Department of Commerce**

**Washington, DC 20230(US)**

Proprietor: **LITTON BIONETICS, INC.**
**2020 Bridge View Drive**
**Charleston, SC 29405(US)**

(72) Inventor: **GALLO, Robert, C.**
**8513 Thornden Terrace**
**Bethesda, MD 20817(US)**
Inventor: **POPOVIC, Mikulas**
**3 Pooks Hill Road, No. 401**
**Bethesda, MD 20814(US)**

SCIENCE, vol. 226, pp. 1165-1171, 7 December 1984; George M. Shaw et al, "Molecular Characterization of Human T-Cell Leukemia (Lymphotropic) Virus Type III in the Acquired Immune Deficiency Syndrome.

SCIENCE, vol. 226, pp. 447-449, 26 October 1984; Jerome E. Groopman et al, "HTLV-III In Saliva of People With AIDS-Related Complex and Healthy Homosexual Men at Risk for AIDS"

SCIENCE, vol. 226, pp. 449-451, 26 October 1984; D. Zagury et al, "HTLV-III in Cells Cultured From Semen of Two Patients With AIDS"

THE LANCET, 9 June 1984, pp. 1253-1256; F. Brun-Vezinet et al, "Detection of IgG Antibodies to Lymphadenopathy-Associated Virus in Patents With AIDS or Lymphadenopathy Syndrome"

THE LANCET, 30 June 1984, pp. 1438-1440; B. Safai et al, "Serioepidemiological Studies of Human T-Lymphotropic Retrovirus Type III in Acquired Immunodeficiency Syndrome"

SCIENCE, vol. 224, pp. 475-477, 4 May 1984; Jean L. Marx, "Strong New Candidate for AIDS Agent"

SCIENCE, vol. 224, pp. 497-500, 4 May 1984; M. Popovic et al, "Detection, Isolation and Continuous Production of Cytopathic Retroviruses (HTLV-III) From Patient with AIDS or Pre-Aids"

SCIENCE, vol. 224, pp. 500-503, 4 May 1984; Robert C. Gallo et al, "Frequent Detection and Isolation of Cytopathic Retroviruses (HTLA-III) From Patients with AIDS or at Risk for Aids"

SCIENCE, vol. 224, pp. 503-505, 4 May 1984; Jorg Schupbach et al, "Analysis of a Subgroup of Human T-Lymphotropic Retroviruses (HTLV-III) Associated with AIDS"

Inventor: SARNGADHARAN, Mangalasseril, G.
2238 Richelieu Drive
Vienna, VA 22180(US)

(74) Representative: Daley, Michael John et al
F.J. CLEVELAND & COMPANY 40/43 Chancery Lane
London, WC2A 1JQ(GB)

SCIENCE, vol. 224, pp. 506-506, 4 May 1984; M.G. Sarngadharan et al, "Antibodies Reactive With Human T-Lymphotropic Retroviruses (HTLV-III) in the Serum of Patients with AIDS"

JOURNAL OF EXPERIMENTAL MEDICINE, vol. 159, pp. 1117-1131, April 1984; Thomas J. Palker et al, "Monoclonal Antibodies Against Human T-Cell Leukemia-Lymphoma Virus (HTLV) p 24 Internal Core Protein"

HAEMATOLOGY AND BLOOD TRANSFUSION, vol. 28, pp. 311-319; R.C. Gallo et al, "Human T-Cell Leukemia-Lymphoma Virus (HTLV): A Progress Report

C.R. SEANCES ACADEMIE SCIENCE (France), Serie III, vol. 297, pp. 195-197, 3 October 1983; Guy de The et al, "Presence d'Anticorps Anti-HTLV (Human T-Cell Leukemia Virus) Chez les Donneurs de Sang de la Martinique"

LABORATORY INVESTIGATION, vol. 49, pp. 371-377, 1983; Carl Saxinger et al, "Application of the Indicrect Enzyme Linked Immunosorbent Assay Microtest to the Detection and Surfeillance of Human T-Cell Leukemia-Lymphoma Virus"

SCIENCE, vol. 220, pp. 806-809, 20 May 1983; Jean L. Marx, "Human T-Cell Leukemia Virus Linked to AIDS"

SCIENCE, vol. 220, pp. 859-862, 20 May 1983; M. Essex et al, "Antibodies to Cell Membrane Antigens Associated with Human T-Cell Leukemia Virus in Patients with AIDS"

SCIENCE, vol. 220, pp. 865-867, 20 May 1983; Robert C. Gallo et al, "Isolation of Human T-Cell Leukemia Virus in Acquired Immune Deficiency Syndrome (AIDS)"

SCIENCE, vol. 226, pp. 451-453, 26 October 1984; David D. Ho et al, "HTLV-III in the Semen and Blood of a Healthy Homosexual Man"

SCIENCE, vol. 225, pp. 840-842, 24 August 1984; Jay A. Levy et al, "Isolation of Lymphocytophatic Retroviruses from San Francisco Patients with AIDS"

SCIENCE, vol. 225, pp. 69-72, 6 July 1984; P. M. Feorino et al, "Lymphadenopathy-Associated Virus Infection of a Blood Donor-Recipient Pair with Acquired Immunodeficiency Syndrome"

SCIENCE, vol. 225, pp. 321-323, 20 July 1984; V.S. Kalyanaraman et al, "Antibodies to the Core Protein of Lymphadenopathy-Associated Virus (LAV) in Patients with AIDS"

MORBIDITY AND MORTALITY WEEKLY REPORT, vol. 33, pp. 377-379, 13 July 1984; D.C. Des Jarlais et al, "Antibodies to a Retrovirus Etiologically Associated with AIDS in Populations with Increased Incidences of the Syndrome"

PROCEEDINGS OF THE NATIONAL ACADEMY OF SCIENCES, vol. 81, pp. 3856-3860, June 1984; T.H. Lee et al, "Human T-Cell Leukemia Virus-Associated Membrane Antigens: Identity of the Majr Antigens Recognized After Virus Infection"

SCIENCE, vol. 226, pp. 453-456, 26 October 1984; F. Brun-Vezinet et al, "Prevalence of Antibodies to Lymphadenopathy-Associated Retrovirus in Africand Patients with AIDS2

## Description

Technical Field

This invention relates to the detection of antibodies in sera of AIDS and pre-AIDS patients and to biochemical and immunological analysis of HTLV-III virus and its antigens.

Background of the Invention

A family of T-lymphotropic retroviruses causes T-cell proliferation leukemia, T-cell depletion, and immunosuppression in humans infected by the viruses. These retroviruses are known as the HTLV family of T4 tropic retroviruses. Subgroup HTLV-I causes T-cell proliferation and leukemia; subgroup HTLV-II induces T-cell proliferation in vitro but its role in disease is unclear. A third group of related virus, collectively designated HTLV-III, has now been isolated from cultured cells of patients with acquired immune deficiency syndrome (AIDS). The biological properties of HTLV-III and immunological analysis of its proteins show that this virus is a member of the HTLV family. Sera of nearly 100% of patients with AIDS and of nearly 90% of homosexual men with pre-AIDS, but less than 1% of heterosexual donors, have antibodies reactive against antigens of HTLV-III. The major immune reactivity appears to be directed against p24, a 24,000 molecular weight protein believed to be a core antigen, and against p41, a 41,000 molecular weight protein believed to be an envelope antigen of the virus.

Acquired immune deficiency syndrome (AIDS) is a relatively recently recognized disease evident in several parts of the world. Its overwhelming prevalence among homosexual men with multiple sexual partners, illegal intravenous drug abusers, hemophiliacs, blood transfusion recipients, and those with close heterosexual contacts with members of the above high-risk groups strongly suggests that the disease spreads by the transmission of an infectious agent. The primary targets of affliction in the human body are specific subpopulations of T-cells. The severe immune deficiency of these patients results from an unusually low proportion of helper T-cells (T4) in their lymphocyte population, thus reducing the availability of many T4 helper functions, among which is the production of antibodies by B-cells.

Retrovirus infection is known to lead to depressed immune functions in animal systems. Analogizing the human response to these non-human systems, a human retrovirus with a tropism for T-cells was considered a candidate in the etiology of human AIDS. As mentioned above, several members of a family of human T-lymphotropic retroviruses (HTLV) have been isolated. One of these isolates was obtained from a black American with an aggressive form of T-cell lymphoma. This virus, designated HTLV-I, has been etiologically linked to the pathogenesis of adult T-cell leukemia/lymphoma (ATLL). In vitro infection with HTLV-I can alter T-cell function and, in some cases, leads to T-cell death. Another member of the HTLV family was isolated from a patient with a T-cell variant of hairy cell leukemia and was designated HTLV-II. Isolation of HTLV-I and HTLV-II have been reported from cultured T-cells of patients with AIDS. (see Science, 20 May 1983, Vol. 220, pages 865-867). Isolation of another retrovirus was reported from a homosexual patient with chronic generalized lymphadenopathy, a syndrome that often precedes AIDS and is therefore referred to as "pre-AIDS." Proviral DNA of HTLV-I was detected in the cellular DNA of two AIDS patients, and sera of some patients were shown to react with antigens of HTLV-I. The correlation between AIDS and serum antibodies to HTLV-I protein is weak.

EP-A- 138 667 discloses means and method for the diagnosis of lymphadenopathy and acquired immune deficiency syndrome. This application teaches the existence and use of a virus and lysates and core antigens thereof. However, the application teaches that its virus is non-reactive with the p24 core proteins of HTLV-I, and that it includes p25 proteins which are not recognized by HTLV-p24 antiserum.

The present invention shows that the primary cause of the syndrome is a human T-lymphotropic retrovirus variant with limited cross reactivities with the known HTLV-subgroups. These new variants are designated HTLV-III. Disclosed is the use of this virus in an immunological screening of sera of patients with AIDS, pre-AIDS, and individuals at increased risk for AIDS.

HTLV-III was purified from supernatants of cell cultures supporting the continuous production of these cytopathic viruses. These HTLV variants (HTLV-III) lack immortalizing (transforming) properties for normal T-cells and mainly exhibit cytopathic effects on the T-cell helper. The cytopathic effect was overcome by finding a highly susceptible, permissive cell for cytopathic variants of HTLV, thus preserving the capacity for permanent growth after infection with the virus. These cell cultures allow for continuous production of the HTLV-III virus.

Disclosure of Invention

It is shown that antigens associated with the infection of human cells by HTLV-III virus are specifically recognized by antibodies from AIDS patients. In particular, HTLV-III isolated from AIDS patients and transmitted by cocultivation with an HT cell line, or its antigens, specifically reacts with antibodies from human sera taken from AIDS patients. Detection of the presence of the antibodies or the antigens, as desired, may be achieved by means of this reaction. This assay is very useful in monitoring HTLV-III virus infected cells and generally the cultures that contain this virus to make sure that they really produce the virus, as well as to detect AIDS antibodies in patients' sera. If there is an unidentified culture, the antigens provide one way to tell if that culture has the virus HTLV-III. A competition radioimmunoassay for the antigens may be performed using the purified antigens of HTLV-III. If the culture material competes in the assay, then it means that the culture has this protein. If it has the protein, then it has the virus because without the virus it cannot have the protein. The detection methods preferred include radioimmunoassay (RIA), ELISA (an enzyme-linked immunoabsorbent assay) and ELIA (an indirect immunofluorescent assay), as well as Western Blot and Western Dot techniques. The p24 and p41 antigens are particularly preferred. The p24 antigen is a major core protein of the virus, while the p41 antigen is a major envelope antigen of the virus.

According therefore to the present invention there is provided a method for detection of a lymphotropic retrovirus, which is termed HTLV-III virus, is normally cytopathic toward its preferential target, helper $T_4$ cells, has a density in sucrose density-banding of 1.16g/ml, has a major core antigen p24 of molecular weight 24,000 and a major envelope antigen gp41 of molecular weight 41,000 and shows only limited cross-reactivity with all other known HTLV-subgroups, recombinant fractions of HTLV-III virus, antibodies to HTLV-III virus or antigens of HTLV-III virus in a sample, characterised in that said sample is subjected to a competition immunoassay discrimination procedure and/or a Western Blot assay procedure, wherein said procedure employs HTLV-III antigens obtained from an immortal cell culture or independently derived antibodies to HTLV-III, said procedure being specific for HTLV-III proteins other than antigens disclosed in European Patent Application No. 138,667 or antibodies thereto.

## Brief Description of Figures

Figure 1 shows the identification of HTLV-III antigens by sera of patients in accordance with the procedure of Example 2.

Figure 2 shows protein bands caused by reaction with rabbit hyperimmune sera to HTLV-I, HTLV-II, and HTLV-III.

Figure 3 shows the SDS-polyacrylamide gel profile of a sample labelled with [125]I.

Figure 4 shows the results of immunoprecipitation of $^{125}$I labelled p24 of HTLV-III with sera of patients with AIDS and AIDS-related conditions.

Figure 5A shows total competition of the precipitation of labelled p24 by unlabelled HTLV-III extract.

Figure 5B shows two cell clones infected with HTLV-III (H4/HTLV-III and H9/HTLV-III) which blocked the immunoprecipitation of HTLV-III p24 and shows that corresponding uninfected cells were totally without effect.

## Statement of Deposit

A cell line related to the present invention and denoted H9/HTLV-III$_B$, has been deposited in the American Type Culture Collection (ATCC, 12301 Parklawn Drive, Rockville, Maryland 20852-1776, USA) under ATCC No. CRL 8543 on April 19, 1984. H9 is a representative and preferred cloned cell line in accordance with the invention. An additional deposit of Molt 3/HTLV-III$_B$ was made in the ATCC on August 15, 1984, under ATCC No. CRL 8602. These deposits assure permanence of the deposits and ready accessibility thereto by the public.

## Modes for Carrying Out the Invention

Lysates of immortalized human T-cell clones such as H9 to which HTLV-III has been transmitted by cocultivation with lymphocytes from AIDS (designated H9/HTLV-III$_B$) were tested with human sera in a strip radioimmunoassay (RIA) based on the Western Blot technique. The sera used for the analysis were also tested by ELISA with purified HTLV-III. Sera from patients with AIDS and from some homosexuals and heroin-addicts recognized a number of specific antigens not detected by any other means. In short, the antigens associated with HTLV-III virus produced by HT cells permit the detection of antibodies in the sera of AIDS and pre-AIDS patients. The HTLV-III virus and its antigens also make possible the detection of AIDS and pre-AIDS in other samples of human sera, such as donated blood.

Also, a DNA fusion has led to the conclusion that there are at least two definite fractions to the HTLV-III, namely HTLV-IIIA and HTLV-IIIB, particularly susceptible to use in the present assay test kit procedure. Additionally, recombinant fractions of HTLV-III have been prepared. These close derivatives of HTLV-III are included within the scope of the present invention.

Prominent immune reactivity or specificity is directed against p41, a 41,000 molecular weight protein which is an envelope antigen of the HTLV-III virus, and against p24, a 24,000 molecular weight protein which is a core antigen of HTLV-III virus. The p24 antigen is called the p30 homolog because in most viruses the molecular weight is about 30,000. In bovine leukemia virus and in the HTLV series the molecular weight of the protein is about 24,000. (In testing proteins to show the relationship by amine acid sequences, the concept of homology is important After determining the amino acid sequences of the proteins, when these sequences are lined up, if the same amino acids appear at the same positions in both proteins, the two proteins are said to have homology). Sera with patients with AIDS, some homosexuals, and heroin addicts also recognize a number of other specific antigens not detected in normal sera. These antigens are about molecular weight 65,000 (p65), 60,000 (p60) and 55,000 (p55). Although other antigens were detected, these were the most significant. Example 4 provides an illustration of the specificity of these reactions.

The HTLV-III virus may be purified from an impure source of the virus, such as the tissue culture fluid, as follows. The virus is concentrated and purified with sucrose density-banding, a procedure described in J. of Virology, 38:906-915, June 1981. The viruses have their characteristics density -- type C retroviruses, of which HTLV-III is a member, have a density of 1.16 grams per cc in a sucrose solution. (The virus fraction may be located by assaying aliquots of each fraction for HTLV-III specific reverse transcriptase activity). The cells are removed from the culture medium, giving a clarified medium. The medium is centrifuged through a continuous flow centrifuge and as the medium is loaded, because of the rotary spinning, the particles inside the sample move towards the periphery of the rotor through a sucrose gradient between 20-60% with a density in the range from 1.08 to 1.29 g/cc. These particles move towards the periphery until they reach the point in the sucrose gradient where they find their own density in the surroundings (about 36%-38% sucrose, which has the same density as about 1.16). They cannot go any farther. When all clarified culture medium has gone through the rotor, all the particles will have collected to that point (band). The centrifugation is continued so the virus reaches an equilibrium, the particles go no farther, and the fractions are collected by pumping out the contents of the rotor and collecting fractions of different densities. Thus, the virus is purified. There are no cells in the 1.16 density fraction because the cells are removed initially, and those that may have escaped the initial removal step go farther down in the sucrose gradient since they are denser than the virus particles. There is little contamination from free proteins from the culture medium because they are lighter. The banded material in the density range of 1.15-1.18 g/cc is collected and used in the experiment.

To obtain the core protein p24, non-ionic detergents such as NP®40 or Triton® X100 or Tween® 20, and salt (sodium chloride) are added. The combination is important because the detergent breaks up the envelope, which also contains lipids. Detergents emulsify the lipid and remove it from the structure. The virus particles are thus accessible to the sodium chloride. The ionic surroundings resulting from the addition of sodium chloride will destroy the interaction of proteins, including the core proteins, with the nucleic acid in the core. A suspension of nucleic acids and free proteins is thus obtained when the virus is treated with the detergent and salt. The ionic strength of the medium is not lowered until the nucleic acids are removed because they could recombine to form a complex. Therefore, the salt is reduced slightly to 0.3M and then passed through a column of an ion exchange resin, diethylaminoethyl cellulose (DEAE cellulose), which is a cationic matrix. It is positively charged so it will bind a negative charge. The virus extract is passed through the column of DEAE cellulose at about 0.3 M salt, which is enough to keep the nucleic acids and proteins apart. When it goes through the column, DEAE-cellulose picks up the nucleic acids, leaving the proteins to exit the column unbound. After the nucleic acids are removed by the DEAE cellulose, the protein solution is dialyzed against a buffer. The pH is adjusted to about 6.5 and then chromatography is performed on phosphocellulose. The protein binds to the column and is eluted with a gradient of salt. Different proteins elute at different salt concentrations. The p24 elutes between 0.25 and 0.35 molar sodium chloride. Alternately, purification may be achieved by high performance liquid chromatography (HPLC).

In summary, the process for purifying and recovering p24 viral antigen of HTLV comprises the following steps:

(1) Removing the cells from tissue culture medium;
(2) Concentrating and purifying the impure virus by sucrose density-banding;
(3) Adding a non-ionic detergent, such as Triton X100, and sodium chloride to free the nucleic acids and proteins inside the virus; and

(4) Chromatographing on DEAE cellulose (positive) and then subsequently chromatographing on phosphocellulose (cellulose phosphate resin).

Radioimmunoassay techniques for detecting antibodies include radiolabeled assays of the so-called blot technique, such as the Western Blot technique exemplified by Example 2, below.

Also operable and preferred for the detection of antibodies are the enzyme-linked immunosorbent assays (ELISA) (see Example 1). The principle of the ELISA test is the reaction of specific antibodies in sera of AIDS patients with antigens of HTLV-III (preferably previously attached to the well surface of a multiwell dish), and a subsequent reaction of the immune complex thus formed (preferably on the dish surface) with a secondary antibody reactive with human immunoglobulins. The secondary antibody is tagged with a suitable enzyme which yields a colored soluble product when incubated with an appropriate substrate. The intensity of the color yielded is a measure of the antibody titer in the patient sera and is measured using an ELISA PLATE READER.

The components of the ELISA test kit are:

1) Microtiter plates coated with HTLV-III lysate and treated with protein solutions to prevent further adsorption of proteins to the plates by means other than antigen-antibody interaction;

2) Control positive human serum of known reactivity and antibody titer to HTLV-III antigens;

3) Negative control serum with no antibody titer to HTLV-III antigens;

4) Secondary antibody reactive against human immunoglobulins, labeled with an enzyme (e.g., peroxidase, phosphatase, etc.), and a diluent solution to dilute the antibody, comprising normal serum of the animal species in which the secondary antibody is raised;

5) A substrate mixture (e.g., 0.05% orthophenylenediamine and 0.005% $H_2O_2$) for peroxidase;

6) A solution to stop the enzyme reaction (e.g., 4N $H_2SO_4$ for the peroxidase reaction with orthophenylene diamine and $H_2O_2$);

7) Washing buffer containing PBS and 0.02% Tween-20 (or another); and

8) Phosphate buffered saline (PBS).

The test is carried out by adding the test serum to the antigen-coated wells of the microtiter plates and incubating at room temperature for 2 hours (or longer). The wells are washed with the wash buffer and incubated with the secondary antibody with the enzyme tag for 1 hour at 37°C. The plates are washed again with wash solution and subsequently with PBS alone. The plates are then treated with the substrate solution and incubated for 10-30 minutes and the reaction stopped by adding the appropriate solution. The color yield in each well is measured by an ELISA PLATE READER.

The ELISA system above can be modified to substitute purified p24 of HTLV-III instead of lysate of HTLV-III.

Antibodies to HTLV-III may also be detected by an indirect immunofluorescence assay. See Example 3 for an example of this technique. This assay is significant because it uses the infected T-cell as a starting material. ELISA and Western Blot techniques start with the HTLV-III virus.

As indicated above, antibodies to HTLV-III may also be detected in sera of patients with AIDS or pre-AIDS by means of the Western Blot technique (see for instance Example 2). HTLV-III is lysed and electrophoretically fractionated on a poly-acrylamide slab gel. Protein bands on the gel are then electrophoretically transferred to a nitrocellulose sheet, for instance by transverse electrophoresis. The remaining protein binding sites on the nitrocellulose sheet are saturated by incubating the sheet in a solution containing an unrelated protein such as bovine albumin, the sheet is separated into narrow strips such that each strip contains a representative profile of the viral antigens as discrete bands, and the individual strips are used as matrix-bound antigens for detecting antibodies reactive with them in serum samples. Strip solid phase radioimmunoassays have been performed. Test sera obtained from human patients suspected of having contracted AIDS are added to tubes containing the above described strips. Another antibody of $^{125}$I labeled goat anti-human immunoglobulin is added to the reaction strips which are then exposed to X-ray film. Strips positive for the presence of AIDS antibodies exhibit wide bands at the 41,000 molecular weight location.

The components of the Western Blot test kit include:

1) Nitrocellulose (or other polymeric matrix) sheets with the viral protein bands already resolved by gel-electrophoresis and transferred to the sheets as noted above and appropriately blocked with protein solutions to prevent further nonspecific protein binding;

2) A positive control human serum;

3) A negative control human serum;

4) A secondary antibody to human immunoglobulin raised in an appropriate animal host and tagged with a radioactive label;

8

5) Diluent solution;

6) A wash solution (0.5% sodium deoxicholate, 0.1M NaCl, 0.5% Triton X-100, 0.1mM phenylmethylsulfonyl fluoride and 10mM sodium phosphate; pH 7.5); and

7) Test tubes with caps (e.g., 15cc).

To use the Western Blot test kit, each strip is put in a 15cc tube containing 2.5 ml of the diluent and treated with 25 μ l of the test serum and incubated at 4° C overnight. The solution is drained and the strip is washed in the same tube with three changes of the wash solution, agitating well during the wash. 2.5 ml of the diluent is added to each tube and incubated for 1 hour at room temperature. To the contents of the tube is added 20 μl of the radioactive secondary antibody solution (approximately $2.5 \times 10^6$ dpm of $^{125}$I). After an additional incubation of 30 minutes at room temperature, the liquid is drained, the strips are again washed as above, the individual strips are removed from the tubes, blotted dry on a paper towel and mounted in order on filter-paper, wrapped with thin plastic film such as that of the Saran Wrap brand, and exposed to X-ray film in the dark. The film is developed after 24-48 hours. The antibodies reactive with the viral antigens are detected by dark images on the X-ray film. Positive antibody reactions to a 40-45,000 molecular weight antigen and a 23-25,000 molecular weight antigen are diagnostic of HTLV-III antibodies.

Purified p24 may be radioactively labeled with $^{125}$I. Iodine is attached primarily to tyrosine residues of the protein. Radiolabeling procedures and radioimmunoassay (RIA) procedures may be performed in accordance with the methods described by Kalyanaraman et al in J. of Virology, 38(3) :906-915, June 1981.

The principle of this test procedure is based on the formation of immune complexes between antigens (generally proteinaceous materials or carbohydrate molecules or other macromolecules) and specific antibodies to the relevant antigens (for instance rabbit Ig against HTLV-III). These complexes are further reacted with a second antibody reactive against the first antibody used (for instance goat Ig against rabbit Ig). This reaction results in the formation of a ternary complex consisting of the antigen, the primary antibody and the secondary antibody. In addition, the primary antibody, which is always used in excess of that needed for complexing all the antigen present in the test, also forms binary complexes with the secondary antibody. The mixture of the complexes appears as a visible precipitate and can be sedimented by centrifugation. The antigen used in the test is radioactively labeled, usually with the isotope $^{125}$I. Therefore, the immune precipitation can be easily monitored by estimating the radioactivity associated with the precipitate by appropriate counting (e.g., by gamma counting for $^{125}$I-labeled antigen).

The components of the competition radioimmunoassay test kit for HTLV-III p24 include:

(1) Labeled p24 (e.g., $^{125}$I);

(2) A primary antibody reactive with HTLV-III p24 (either hyperimmune antibody raised in an animal by inoculation with an antigen preparation containing HTLV-III p24, or a patient serum known to contain precipitating antibody against HTLV-III p24);

(3) A secondary antibody against the primary antibody (e.g., antibody against rabbit immunoglobulins if the primary antibody used is a rabbit immunoglobulin, or antibody against human immunoglobulin if the primary antibody is a human serum reactive against HTLV-III p24);

(4) The test material to be evaluated for the presence of HTLV-III p24;

(5) pH buffers, surfactants, etc.

The procedure utilizing this test kit involves incubation of multiple dilutions of the non-radioactive test material with the primary antibody in separate test tubes for 1 hour prior to adding a constant amount of the radioactive antigen. The reaction mixture is then incubated initially at 37° C and subsequently at 4° C overnight. The secondary antibody is then added and the reaction mixture further incubated for 1 hour at 37° followed by 2 hours at 4° C. The incubation mixture was then centrifuged to sediment the precipitate, the supernatant fluid aspirated out and the radioactivity in the precipitate determined using a gamma counter.

The radioactivity in the precipitate, in the reaction mixture not containing a competing antigen, is taken as the maximum precipitation of the labeled antigen. When preincubation of the primary antibody with an unlabeled antigen causes a reduction in the radioactivity associated with the precipitate, the conclusion is that the unknown test sample contains antigenic activity of HTLV-III p 24.

Detailed Description of the Figures

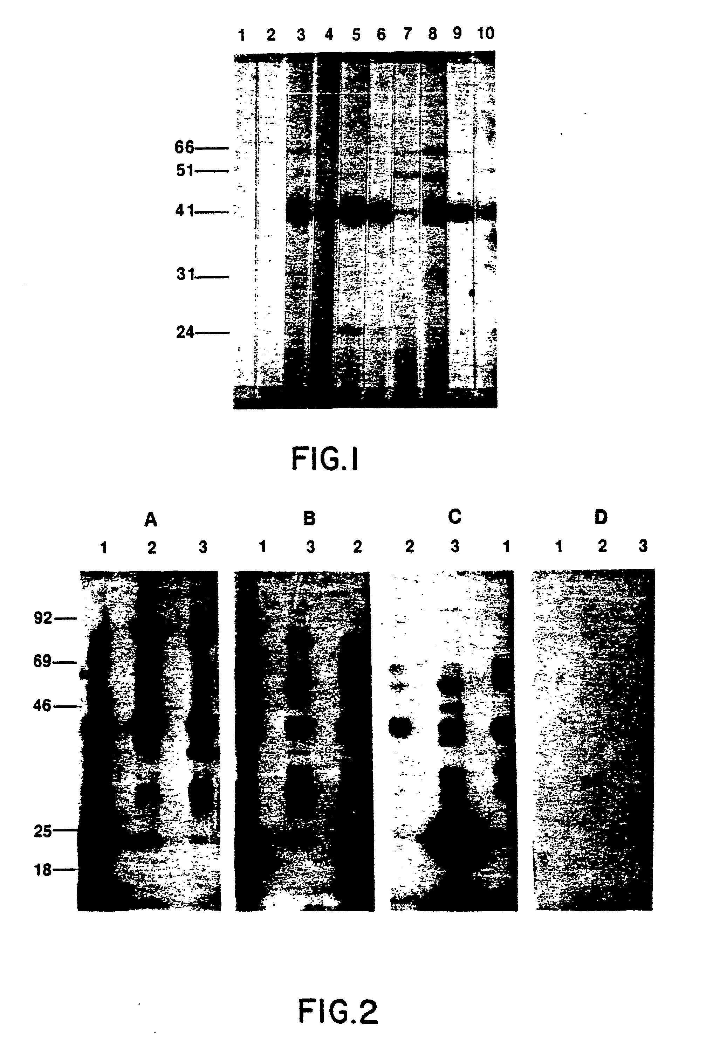

Figure 1 shows the identification of HTLV-III antigens by sera patients in accordance with the procedure of Example 2.

Figure 2 is a Western Blot analysis of antigenic cross-reactivities among HTLV-I, HTLV-II, and HTLV-III. Extracts of HTLV-I, HTLV-II, and HTLV-III were fractionated by SDS-polyacrylamide gel electrophoresis and the protein bands electroblotted to a nitrocellulose sheet. After blocking the remaining protein binding sites by incubation with 5% bovine serum albumin, the sheet was cut into segments, each containing lanes of HTLV-I, HTLV-II, and HTLV-III proteins. Segment A was incubated with a rabbit antiserum prepared against HTLV-I extract, segment B with a rabbit serum against HTLV-II extract, segment C with rabbit serum against HTLV-III extract and segment D with normal rabbit serum. After washing, the segments were reacted with 125I-labeled goat IgG against rabbit immunoglobulin. The sheets were thoroughly washed, blotted dry and exposed to X-ray film. Lanes 1, 2, and 3 in each segment represent HTLV-I, HTLV-II, and HTLV-III. Numbers on the left represent molecular weights in thousands of standard marker proteins co-electrophoresed.

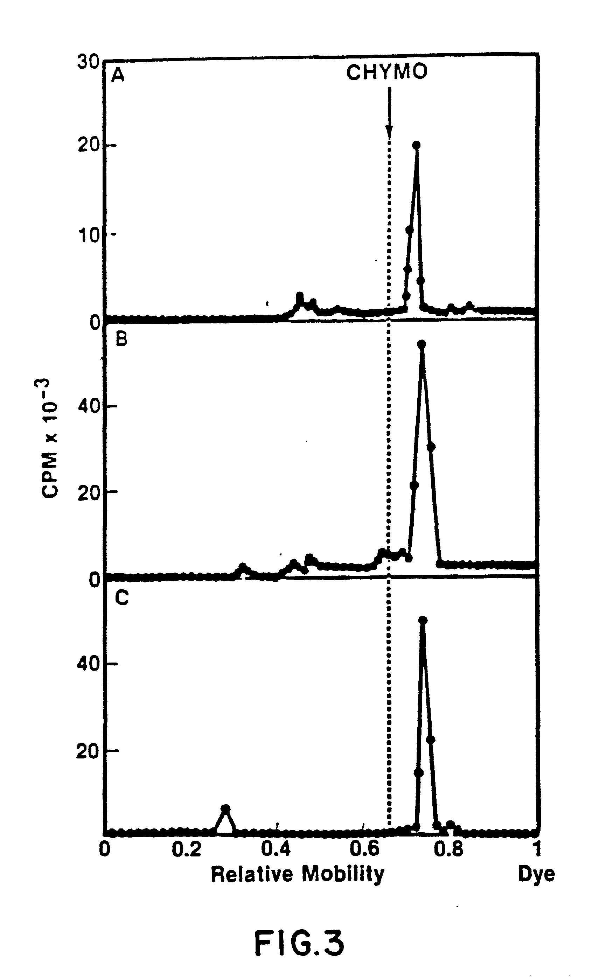

Figure 3 shows electrophoretic profiles of 125I-labeled p24 from HTLV-I, HTLV-II, and HTLV-III. 125I-labeled p24 proteins of HTLV-I (C), HTLV-II (B), and HTLV-III (A) were analyzed by electrophoresis through cylindrical 12% polyacrylamide gels in the presence of SDS. The gels were divided into 1 mm slices and counted in a gamma counter. The position of an 125I-labeled sample of chymotrypsinogen (mwt = 25,500) in a parallel gel is indicated by the vertical dashed line.

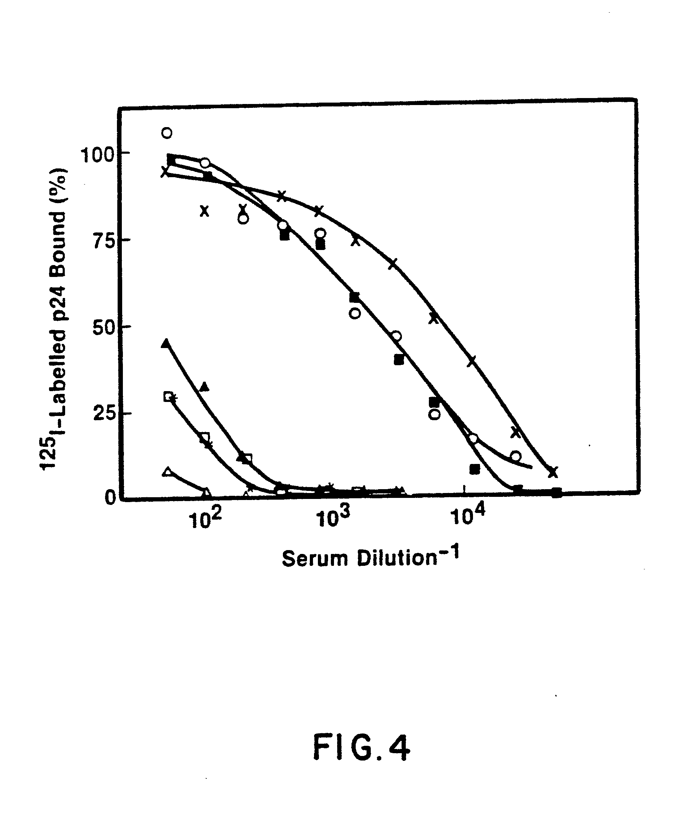

Figure 4 shows the immunoprecipitation of 125I-labeled p24 of HTLV-III by sera of AIDS patients. Immunoprecipitations were performed by the double antibody method of Kalyanaraman et al, J. Virol., 38:906-915 (1981). Serial dilutions of sera were incubated with the labeled p24 of HTLV-III (approximately 8000 cpm) for 3 hours at 37°C followed by overnight at 4°C in a 200 $\mu$l reaction mixture containing 20 mM Tris-HCl (pH 7.5), 0.2 M NaCl, 0.3% Triton X-100, 2 mg/ml of bovine serum albumin, 1 mM EDTA and 0.1 mM PMSF. A 30-fold excess of goat anti-human IgG was then added and the incubation continued for 1 hour at 37°C and 2 hours at 4°C. The incubation mixture was diluted to 1 ml with the buffer and centrifuged at 4,000 rpm for 15 minutes in a Beckman TJ-6 centrifuge. The radioactivity bound in pellet was determined using a gamma counter.

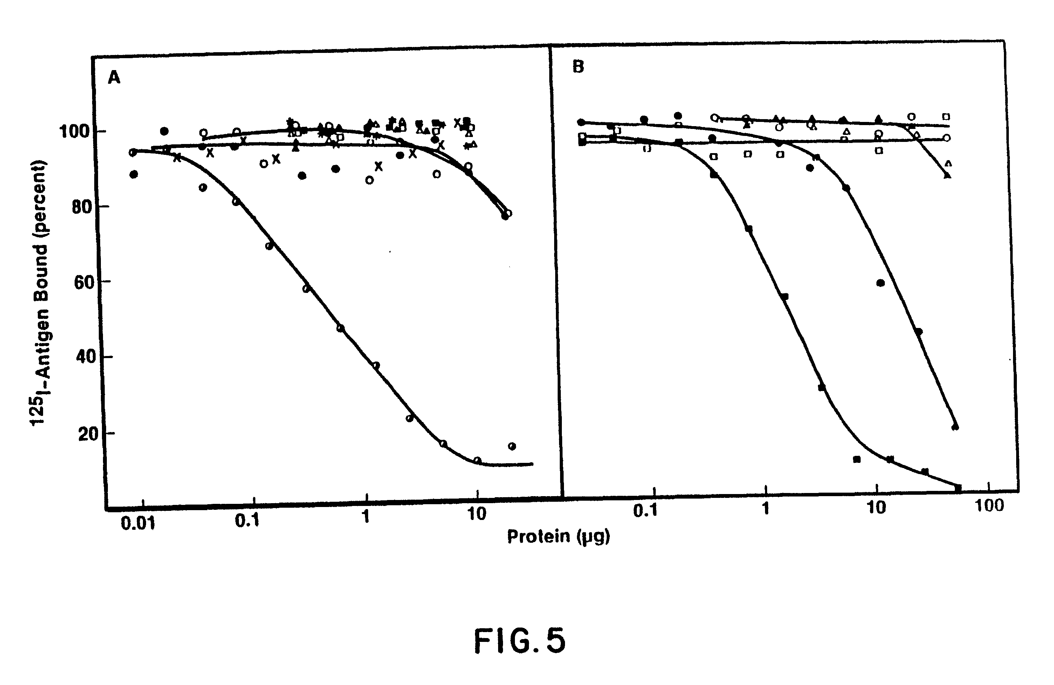

Figure 5 shows the homologous competition radioimmunoassay for HTLV-III p24. The assay used a 1:1500 dilution of a hyperimmune rabbit serum prepared against disrupted HTLV-III. In the absence of competing unlabeled antigens, this serum dilution precipitated 20% of the 125I-labeled p24. The rabbit serum was preincubated for 1 hour at 37°C with the unlabeled antigens listed, prior to adding the labeled antigen (approximately 8000 cpm). Further details of the assay are the same as described in the Figure 4 legend, except that the second antibody used was goat antirabbit immunoglobulin.

A. Competition with retroviral extracts:

o—o, HTLV-III; ●—●, HTLV-I; O—O, HTLV-II; ▲—▲, FeLV; △—△, R-MuLV; ■—■, SSV; □—□, BaEV (M7); ▽—▽ SMRV; ▼—▼, MPMV; *—*, BLV; and x—x, EIAV p26.

B. Competition with cellular extracts:

●—●, H4 cells infected with HTLV-III; o—o, uninfected H4 cells; □—□, uninfected H9 cells; ▲—▲, HUT 102 cells producing HTLV-I; and △—△, C3-44 cells producing HTLV-II.

## Example 1

Antibodies to HTLV-III in Sera of Patients with AIDS and pre-AIDS Lymphadenopathy Syndrome

Wells of 96-well plates were coated overnight with a lysate of density-banded HTLV-III at 0.5 $\mu$g protein per well in 100 $\mu$l 50 mM sodium bicarbonate buffer, pH 9.6. The wells were washed with water and incubated for 20 min. with 100 $\mu$l of 5% bovine serum albumin in phosphate buffered saline (PBS). After washing, 100 $\mu$l of 20% normal goat serum in PBS were added to each well, followed by 5 or 10 $\mu$l of the test sera (blood taken from a human patient), and allowed to react for 2 hours at room temperature. The wells were washed three times with 0.5% Tween-20 in PBS in order to remove unbound antibodies, and were incubated for 1 hour at room temperature with peroxidase labeled goat anti-human IgG at a dilution of 1:2000 in 1% normal goat serum in PBS. Goat anti-human IgG is a second antibody that binds with the antibody-antigen complex formed in positive wells. The wells were successively washed 4 times with 0.05% Tween 20 in PBS and 4 times with PBS to remove unbound goat antibody, and reacted with 100 $\mu$l of the substrate mixture containing 0.05% orthophenylene diamine and 0.005% hydrogen peroxide in phosphate-citrate buffer, pH 5.0. This substrate mixture detects the peroxidase label and forms a colored product. The reactions were stopped by the addition of 50 $\mu$l of 4N $H_2SO_4$ and the color yield measured using an ELISA reader which quantifies the color reading. Assays were performed in duplicate; absorbance readings greater than three times the average of 4 normal negative control readings were taken as positive. The results are shown in Table 1.

## Table 1

| Subjects | No. Positive/No. Tested | Percent Positive |
|---|---|---|
| Patients with AIDS | 43/49 | 87.8 |
| Pre-AIDS | 11/14 | 78.6 |
| Intravenous Drug abusers | 3/5 | 60 |
| Homosexual men | 6/17 | |
| Sexual contact with AIDS patient | 1/1 | |
| Persistent fatigue | 1/1 | |
| Other | 4/15 | 26.6 |
| Other controls | 1/186 | 0.5 |
| Normal subjects | 1/164 | 0.6 |
| Patients with Hepatitis B virus infection | 0/3 | 0 |
| Patients with Rheumatoid arthritis | 0/1 | 0 |
| Patients with Systemic lupus erythematosus | 0/6 | 0 |
| Patients with acute mononucleosis | 0/4 | 0 |
| Patients with lymphatic leukemias | 0/8 | 0 |

### Example 2

Western Blot analysis of the test area was conducted as follows. HTLV-III was lysed and fractionated by electrophoresis on a 12% polyacrylamide slab gel in the presence of sodium dodecylsulfate (SDS). The protein bands on the gel were electrophoretically transferred to a nitrocellulose sheet, according to the procedure of Towbin et al., Proc. Natl. Acad. Sci. USA, 76:4350 (1979). Strip solid phase radioimmunoassays were then performed. The sheet was incubated at 37° for 2 hours with 5% bovine serum albumin in 10 mM Tris-HCl, pH 7.5 containing 0.9% NaCl and cut into 0.5 cm strips. Each strip was incubated for 2 hours at 37° and 2 hours at room temperature in a screw cap tube containing 2.5 ml of buffer 1 (20 mM Tris-HCl, pH 7.5, 1 mM EDTA, 0.2 M NaCl, 0.3% Triton X-100 and 2 mg/ml bovine serum albumin and 0.2 mg/ml of human antibody fractions, Fab). Test sera (25 $\mu$l), taken from human patients with AIDS or exhibiting pre-AIDS symptoms, were then added to individual tubes containing the strips and incubation continued for 1 hour at room temperature and overnight in the cold. The strips were washed three times with solution containing 0.5% sodium deoxycholate, 0.1 M NaCl, 0.5% Triton X-100, 1 mM phenylmethylsulfonyl fluoride and 10 mM sodium phosphate, pH 7.5. The strips were incubated for 1 hour at room temperature with 2.4 ml of buffer 1 and 0.1 ml of normal goat serum. Affinity purified and $^{125}$I-labeled goat anti-human immunoglobulin ( $\mu$ chain and Fc fragment) (1.25 x $10^6$ cpm) were added to the reaction mixture and the incubation continued for 30 minutes at room temperature. The strips were washed as described, dried, mounted and exposed to X-ray film. Figure 1 indicates graphically the results of these experiments. Strip 1 is test sera from an adult with T-cell leukemia; Strip 2 is a normal donor; Strip 3 is a mother of a child with AIDS; Strips 4 and 6-10 are AIDS patients; and Strip 5 is a patient with pre-AIDS.

### Example 3

EP 0 181 374 B1

Fixed Cell and Live Cell Indirect Immunofluorescence Assay for Antibodies to HTLV-III

Indicator cell: HTLV-III infected negative cells; negative control: uninfected T-cells.

Infected cells were washed with phosphate buffered saline (PBS) and resuspended in PBS at $10^6$ cells/ml. Approximately 50 ml of cell suspension were spotted on a slide, air dried, and fixed in acetone for 10 minutes at room temperature. Slides were stored at -20° C until ready for use. 20 ml of the test human serum diluted 1:10 in PBS were added to the fixed cells and incubated for 1 hour at 37°. Slides were washed and reacted for 30 minutes at room temperature with a dilute solution of fluorescein-conjugated goat anti-human IgG. Slides were again washed and examined under a microscope for fluorescence. A negative control used uninfected parental cells. The above describes a fixed cell system in which the antibody antigen reaction is sought for both inside and outside the cell.

For live cell immunofluorescence assay all the above reactions were carried out in a tube instead of on a slide, but without chemical fixation of the cells. After reaction with the fluorescein conjugated anti-human antibody, the cells were added to the slide and examined under a microscope for antibody-antigen reaction on the surface of the cell.

The results of each of these assays show a strong fluorescence reaction specifically with sera of AIDS and pre-AIDS patients.

Example 4

Sera of patients with clinically documented AIDS, Kaposi's sarcoma, sexual contacts with AIDS patients, intravenous drug abusers, homosexual men and heterosexual donors were tested for their reactivity to HTLV-III. The system employed was ELISA. Lysates of sucrose density banded HTLV-III were coated on 96-well microtiter plate wells. Test sera were diluted with a dilute solution of normal goat serum, added to the wells, and allowed to react for 2 hours or overnight at room temperature. The antibodies in the human sera were detected by the reaction of the primary immune complex with peroxidase labeled goat anti-human immunoglobulins followed by the development of a colored peroxidase reaction product. The results obtained are presented in Table 1. Of 49 clinically diagnosed AIDS patients, 43 (88%) showed serum reactivity in this assay. Fourteen homosexual men with pre-AIDS were also tested for anti-bodies to HTLV-III. Of these, II (79%) were positive. Among 17 homosexual men with no clinical symptoms of AIDS, 7 scored positive. Of these, at least one was known to be a long-time sexual partner of a diagnosed case of AIDS. One had persistent fatigue and was exhibiting early signs of AIDS. One of the three intravenous drug abusers that were positive for serum anti-bodies to HTLV-III was also homosexual.

In contrast, only 1 of 186 controls tested reacted positive in this test. They included 3 with hepatitis B virus infection, 1 with rheumatoid arthritis, 6 with systemic lupus erythematosus, 4 with acute mononucleosis and 8 with various forms of lymphatic leukemias and lymphomas, some of whom were positive for HTLV-I. The remaining test subjects were normal donors of unknown sexual preference, including laboratory workers ranging in age from 22 to 50.

Example 5

To investigate the specificity of the reactions, lysates of the HTLV-III-infected cell clones were analyzed in comparison with lysates of the same cell clones before viral infection. No reactive antigen was found in the uninfected clones, with the exception of an 80,000 molecular weight band in H17 which bound antibodies from all human sera tested, but not from rabbit or goat serum. Antigens newly expressed after viral infection and recognized by the human serum used for this analysis include p65, p55, p41, p39, p32 and p24. In addition, a large protein of approximately 130,000 molecular weight and one of 48,000 molecular weight were detected. With normal human serum, none of the antigens was detected. These results show that the antigens detected are either virus-coded proteins or cellular antigens specifically induced by viral infection.

Example 6

Sucrose density banded HTLV-III from culture supernatants of H9/HTLV-III cells was analyzed by the Western blot technique for determination of its antigenic similarities with HTLV-I and HTLV-II. Lysates of HTLV-I, HTLV-II, and HTLV-III were fractionated by SDS-polyacrylamide gel electrophoresis and electroblotted to a nitrocellulose sheet. The protein bands were then reacted with rabbit hyperimmune sera to HTLV-I, HTLV-II, or HTLV-III. The results are shown in Figure 2. The strongest immune reactivity for p24 was observed with the homologous antisera (Lanes A-1, B-2, and C-3). Moderately strong cross-reactivities were seen between HTLV-II p24 and anti-HTLV-I (Lane A2), and HTLV-III p24 and anti-HTLV-II (Lane B3). Weak reactivities were also seen between HTLV-III p24 and anti-HTLV-I (Lane A3), and HTLV-I p24 and an anti-HTLV-III (Lane C1). However, in spite of the moderate reactivity between HTLV-III p24 and anti-HTLV-II (Lane B3), no reciprocal reactivity between HTLV-II p24 and anti-HTLV-III (Lane C2) was found. No reactivity was observed with normal rabbit sera (Panel D).

The major HTLV-III core protein, p24, was purified from the lysate of a sucrose density banded virus preparation. The virus was solubilized with 0.5% Triton X-100, 0.8M NaCl, 20% glycerol and 0.1 mM phenylmethylsulfonyl fluoride (PMSF) in 50 mM Tris-HCl, pH 7.9, and the extract was freed of nucleic acids by a batch adsorption and elution from DEAE-cellulose with 0.3M NaCl. The eluate was dialyzed against 50 mM BES buffer, pH 6.5 containing 1 mM EDTA, 0.1 mM PMSF and chromatographed on a phosphocellulose column equilibrated with the same buffer. The proteins were eluted using a linear 0 to 0.6 M NaCl gradient. HTLV p24 eluted between 0.25 and 0.3 M NaCl. Figure 3 shows the SDS-polyacrylamide gel profile of a sample after labeling with $^{125}$I. In electrophoretic mobility, HTLV-III p24 was very similar to HTLV-I p24 and HTLV-II p24, and significantly faster than $^{125}$I-labeled chymotrypsinogen run in a parallel gel as a molecular weight marker (mwT = 25,500) (Figure 2, vertical dashed line).

The specificity of the purified p24 was analyzed by immunoprecipitation with patient sera and by competition radioimmunoassays using hyperimmune rabbit sera.

Figure 4 shows the results of immunoprecipitation of $^{125}$I-labeled p24 of HTLV-III with sera of patients with AIDS and AIDS related conditions. A wide range of antibody titers was evident from these experiments and this finding agreed with results from Western Blot analysis that indicated that immune reactivities with p24 tended to vary widely and that sera of some AIDS patients did not show significant antibody reactivity with p24.

A homologous competition radioimmunoassay was set up using $^{125}$I-labeled HTLV-III p24 and a rabbit antibody raised against disrupted HTLV-III. Figure 5A shows a total competition of the precipitation of the labeled p24 by unlabeled HTLV-III extract. Extracts of HTLV-I and HTLV-II showed only a minimal competition at protein concentrations about 100-fold higher than HTLV-III. On the other hand, a large number of other mammalian retroviruses (Feline leukemia virus (FeLV), Rauscher murine leukemia virus (R-MuLV), simian sarcoma virus (SSV), baboon endogenous virus (BaEV), squirrel monkey retrovirus (SMRV), Mason-Pfizer monkey virus (MPMV), and bovine leukemia virus (BLV)) did not compete in this immunoprecipitation. Similarly, two cell clones infected with HTLV-III (H4/HTLV-III and H9/HTLV-III) blocked the immunoprecipitation of HTLV-III p24, but the corresponding uninfected cells were totally without effects (Fig. 5B). Cells producing HTLV-I and HTLV-II exhibited marginal effects in this competition at relatively higher protein concentrations. Therefore, the antigens involved in the above reactions were specific to HTLV-III and cells producing HTLV-III. There were detectable, although low level, cross-reactivities between HTLV-III p24 and p24 of HTLV-I and HTLV-II. These cross-reactivities became more easily evident in a less stringent system such as Western Blots (Figure 2). Purified p26 of equine infectious anemia virus (EIAV) here showed little cross-reactivity in the homologous radioimmunoassay for HTLV-III p24 (Figure 5A). The effect of HTLV-III was checked in homologous competition radioimmunoassays for HTLV-I p24 and HTLV-II p24. While these systems showed considerable cross-reactivity between HTLV-I and HTLV-II, competition by HTLV-III was only minimal in either assay.

The results showed that HTLV-III is a unique retrovirus with a major core protein, p24, unrelated to most other retroviruses. The p24 of HTLV-III, however, shares a low but detectable level of antigenic cross-reactivity with HTLV-I and HTLV-II and not with other retroviruses. These cross-reactivities with HTLV-I and HTLV-II were seen more clearly in the Western Blot analysis than in conventional competition radioimmunoassays. It was analogous to the detection of cross-reactivities between HTLV-I p24 and BLV p24 by the Western Blot, although no such cross-reactivities could be demonstrated between the two viruses using competition radioimmunoassays. It was clear that HTLV-I and HTLV-II were more closely related to each other than HTLV-III was to either HTLV-I or HTLV-II. These findings were in agreement with the detection of a limited nucleic acid sequence homology between HTLV-III and both HTLV-I and HTLV-II, especially in the gag-pol region of the genomes.

Example 7

Therapeutic AIDS-specific test kits were constructed for detecting antibodies using several different techniques for detection. One test kit for antibodies detection comprised a compartmented enclosure containing a plurality of wells, plates which were coated prior to use with p24 of HTLV-III and ELISA materials for enzyme detection consisting of normal goat serum and peroxidase-labeled goat antihuman IgG

EP 0 181 374 B1

and a color change indicator consisting of orthophenylene diamine and hydrogen peroxide in phosphate citrate buffer.

A second test kit for detecting antibodies using the Dot Blot technique comprised a container, a cover, and therein containing a nitrocellulose sheet with HTLV p24 attached by "dot-blotting," and additionally surfactants as well as pH modifiers and bovine serum albumin and human Fab. This Dot Blot analysis container also contained a supply of dilute normal goat serum and $^{125}$I labeled goat antihuman immunoglobulin.

Finally, a different AIDS-specific test kit for detecting HTLV-III using competition radioimmunoassay comprised a compartmented container $^{125}$I-labeled p24 of HTLV-III, rabbit (or goat) anti-HTLV-p24, solutions of surfactants, pH buffers, bovine serum albumin and goat antiserum to rabbit immunoglobulin (or rabbit antiserum to goat immunoglobulin).

## Claims

1. A method for detection of a lymphotropic retrovirus, which is termed HTLV-III virus, is normally cytopathic toward its preferential target, helper $T_4$ cells, has a density in sucrose density-banding of 1.16g/ml, has a major core antigen p24 of molecular weight 24,000 and a major envelope antigen p41 of molecular weight 41,000 and shows only limited cross-reactivity with all other known HTLV-subgroups, recombinant fractions of HTLV-III virus, antibodies to HTLV-III virus or antigens of HTLV-III virus in a sample, characterised in that said sample is subjected to a competition immunoassay discrimination procedure and/or a Western Blot assay procedure, wherein said procedure employs HTLV-III antigens obtained from an immortal cell culture or independently derived antibodies to HTLV-III, said procedure being specific for HTLV-III proteins other than antigens disclosed in European Patent Application No. 138,667 or antibodies thereto.

2. A method for detection of a lymphotropic retrovirus, according to claim 1 characterised in that said sample is subjected to a competition immunoassay discrimination procedure and/or a Western Blot assay procedure, specific for an HTLV-III protein selected from p24, p32, p39, p41, p48, p55, p60, p65 and p130.

3. A method according to claim 2, characterised in that said HTLV-III protein is p24.

4. A method according to claim 2, characterised in that said HTLV-III protein is p41.

5. A method according to claim 2, characterised in that said HTLV-III protein is p130.

6. A method according to claim 1, characterised in that HTLV-III virus is used to detect antibodies to HTLV-III virus in said sample.

7. A method according to any of claims 1 to 5, characterised in that an antigen of HTLV-III virus is used to detect antibodies to HTLV-III virus in said sample.

8. A method according to any of claims 1 to 5, characterised in that labelled HTLV-III virus, labelled recombinant fractions of HTLV-III virus or labelled antigens of HTLV-III virus are used in said method.

9. A method according to any of claims 1 to 5, characterised in that labelled antibodies to HTLV-III virus are used in said method.

10. A method according to claim 8 or 9, characterised in that HTLV-III virus, recombinant fractions of HTLV-III virus or antigens of HTLV-III virus in said sample are detected by said method.

11. A method according to any of claims 1 to 7, characterised in that said discrimination procedure is selected from ELISA, immunoprecipitation and immunofluorescence assay methods.

12. A method according to any of claims 1 to 5, characterised in that an antibody to HTLV-III virus is reacted with an antigen of HTLV-III virus to form an immune complex, and said immune complex is reacted with a labelled secondary antibody to produce a readily quantifiable product.

14

**13.** A method according to claim 12, characterised in that said secondary antibody is tagged with an enzyme which can produce a coloured product.

**14.** A method according to claim 12, characterised in that said secondary antibody is tagged with radioactive [125]I.

**15.** A method according to any of claims 1 to 5, characterised in that said discrimination procedure is of the Western Blot type wherein protein bands of HTLV-III virus are formed by electrophoresis of HTLV-III virus on a polyacrylamide gel in the presence of sodium dodecylsulfate, the protein bands in the gel are transferred to a nitrocellulose sheet, the sheet is saturated with an unrelated protein, the sheet is separated into strips representing the profile of the antigens to HTLV-III virus, and the antigens are used for detecting antibodies in said sample.

**16.** A method according to any of claims 1 to 5, characterised in that said antigen is obtained by concentrating HTLV-III virus by ultracentrifugation from virus culture supernatants, removing lipids by centrifugation through 20% (W/W) sucrose in TNE buffer to form a sucrose gradient, dividing the sucrose gradient into fractions, and isolating antigen bands by assaying aliquots of each fraction for HTLV-III virus specific reverse transcriptase activity.

**17.** A method according to claim 8 or 9, characterised in that said sample comprises tissue culture material or primary cells of body fluids.

**18.** A method according to claim 2, characterised in that said envelope antigen p41 is labelled.

**19.** A method according to claim 2, characterised in that said core antigen p24 is labelled.

**20.** An AIDS specific test kit comprising a compartmented enclosure, characterised in that at least one compartment of said enclosure contains at least one of a lymphotropic retrovirus, termed HTLV-III virus, which is normally cytopathic toward its preferential target, helper $T_4$ cells, has a density in sucrose density-banding of 1.16g/ml, has a major core antigen p24 of molecular weight 24,000 and a major envelope antigen p41 of molecular weight 41,000 and shows only limited cross-reactivity with all other known HTLV-subgroups, recombinant fractions of HTLV-III virus, antibodies to HTLV-III virus and antigens of HTLV-III virus;

characterised in that at least one compartment of said enclosure contains at least one HTLV-III protein obtained from an immortal cell culture or an independently derived antibody to HTLV-III proteins, said at least one HTLV-III protein excluding proteins disclosed in EP-A-138,667.

**21.** An AIDS specific test kit according to claim 17, characterised in that at least one compartment of said enclosure contains an HTLV-III protein selected from p24, p32, p39, p41, p48, p55, p60, p65 and p130.

**22.** A test kit according to claim 21, characterised in that said HTLV-III protein is p24.

**23.** A test kit according to claim 21, characterised in that said HTLV-III protein is p41.

**24.** A test kit according to claim 21, characterised in that said HTLV-III protein is p130.

**25.** A test kit according to any of claims 20 to 24, characterised in that said at least one compartment contains antibodies to HTLV-III virus.

**26.** A test kit according to any of claims 20 to 24 characterised in that said at least one compartment contains HTLV-III virus.

**27.** A test kit according to any of claims 20 to 24, characterised in that said at least one compartment contains antigens of HTLV-III virus.

**28.** A test kit according to any of claims 20 to 24, characterised in that said at least one compartment contains HTLV-III virus lysate.

29. A test kit according to claim 28, characterised in that it includes

(a) microtiter plates coated with HTLV-III virus lysate and treated with protein solutions to prevent further adsorption of proteins to the plates by means other than antigen-antibody interaction;

(b) control positive human serum of known reactivity and antibody titer to antigens of HTLV-III virus;

(c) negative control serum with no antibody titer to antigens of HTLV-III virus;

(d) enzyme-labelled secondary antibody reactive against human immunoglobulins, and a diluent solution for the secondary antibody consisting of normal serum of the animal species in which the secondary antibody is raised;

(e) a substrate mixture of orthophenylenediamine and $H_2O_2$;

(f) a solution to stop the enzyme reaction;

(g) washing buffer; and

(h) phosphate buffered saline.

30. A test kit according to claim 26, characterised in that said enclosure contains a plurality of wells, plates which are coated prior to use with HTLV-III and ELISA materials for enzyme detection consisting of normal goat serum and peroxidase, labelled goat antihuman IgG and a colour change indicator.

31. A test kit according to claim 30, characterised in that said colour change indicator consists of orthophenylenediamine and hydrogen peroxide in phosphate citrate buffer.

32. A test kit according to claim 31, characterised in that said HTLV-III is present in the form of a lysate.

33. A test kit according to claim 26, characterised in that said enclosure further contains phosphate buffered saline and fluorescein-conjugated goat antiserum IgG.

34. A test kit according to claim 26, characterised in that said enclosure further contains a nitrocellulose sheet and a polyacrylamide slab gel in the presence of sodium dodecylsulfate, surfactants, pH modifiers, bovine serum albumin, human Fab, and a supply of dilute normal goat serum and $^{125}$I labelled goat antihuman immunoglobulin.

35. A test kit according to claim 27, characterised in that said enclosure contains

(a) nitrocellulose sheets with HTLV-III protein bands already resolved by gel-electrophoresis and appropriately blocked with protein solutions to prevent further nonspecific protein binding;

(b) a positive control human serum;

(c) a negative control human serum;

(d) a secondary antibody to human immunoglobulin raised in an appropriate animal host and tagged with a radioactive label;

(e) diluent solution;

(f) wash solution;

(g) test tubes with caps.

36. A test kit according to claim 35, characterised in that the wash solution is 0.5% sodium deoxicholate, 0.1M NaCl, 0.5% Triton® X-100, 0.1mM phenylmethylsulfonyl fluoride and 10mM sodium phosphate; pH 7.5.

37. A test kit according to claim 20, characterised in that at least one compartment of said enclosure contains the envelope antigen p41.

38. A test kit according to claim 21, characterised in that at least one compartment of said enclosure contains the core antigen p24.

39. A test kit according to claim 20, characterised in that said enclosure contains (a) nitrocellulose sheets with HTLV-III protein bands already resolved by gel-electrophoresis and appropriately blocked with protein solutions to prevent further nonspecific protein binding;

(b) a positive control human serum;

(c) a negative control human serum;

(d) a secondary antibody to human immunoglobulin raised in an appropriate animal host and tagged with a radioactive label;

(e) diluent solution;
(f) wash solution;
(g) test tubes with caps.

**40.** A test kit according to claim 39, characterised in that the wash solution is 0.5% sodium deoxicholate, 0.1M NaCl, 0.5% Triton® X-100, 0.1mM phenylmethylsulfonyl fluoride and 10mM sodium phosphate; pH 7.5.

**41.** A test kit according to claim 39, characterised in that said enclosure further contains primary antibodies reactive either with said envelope antigens p41, or with said core antigen p24; secondary antibodies against the primary antibodies, pH buffers and surfactants.

**42.** A test kit according to claim 37, characterised in that said enclosure contains a reaction vessel for addition of test material to be evaluated for envelope antigens p41, or core antigen p24.

**Revendications**

**1.** Procédé pour détecter dans un échantillon un rétrovirus lymphotropique, désigné sous le nom de virus HTLV-III, qui est normalement cytopathogène à l'égard de sa cible préférentielle, à savoir les cellules inductrices $T_4$, qui a une densité de 1,16 g/ml, déterminée par gradient de densité dans une solution de saccharose, qui a un antigène principal p24 de nucléocapside, ayant un poids moléculaire de 24 000, et un antigène principal p41 d'enveloppe, ayant un poids moléculaire de 41 000, et qui présente une réactivité croisée limitée avec tous les autres sous-groupes de HTLV, et pour détecter des fractions recombinantes du virus HTLV-III, des anticorps antivirus HTLV-III, ou des antigènes du virus HTLV-III, caractérisé en ce que ledit échantillon est soumis à un protocole de discrimination par immuno-essai par compétition et/ou à un protocole d'essai Western Blot, au cours desquels on utilise des antigènes HTLV-III, obtenus à partir d'une culture de cellules immortelles, ou à partir d'anticorps anti-HTLV-III, ledit protocole étant spécifique des protéines HTLV-III (autres que les antigènes décrits dans la demande de brevet européen publiée sous le N° 138,667), ou des anticorps anti-protéines HTVL-III.

**2.** Procédé pour détecter un rétrovirus lymphotropique selon revendication 1, caractérisé en ce que l'échantillon est soumis au protocole de discrimination par immuno-essai par compétition, et/ou au protocole d'essai Western Blot, spécifiques d'une protéine HTLV-III, choisie parmi les p24, p32, p39, p41, p48, p55, p60, p65 et p130.

**3.** Procédé selon la revendication 2, caractérisé en ce que la protéine HTLV-III est la p24.

**4.** Procédé selon la revendication 2, caractérisé en ce que la protéine HTLV-III est la p41.

**5.** Procédé selon la revendication 2, caractérisé en ce que la protéine HTLV-III est la p130.

**6.** Procédé selon la revendication 1, caractérisé en ce que le virus HTLV-III est utilisé pour détecter dans ledit échantillon les anticorps anti-virus HTLV-III.

**7.** Procédé selon l'une quelconque des revendications 1 à 5, caractérisé en ce qu'un antigène du virus HTLV-III est utilisé pour détecter dans ledit échantillon les anticorps anti-virus HTLV-III.

**8.** Procédé selon l'une quelconque des revendications 1 à 5, caractérisé en ce que le virus HTLV-III marqué, des fractions recombinantes marquées du virus HTLV-III, ou des antigènes marqués du virus HTLV-III sont utilisés dans ledit procédé.

**9.** Procédé selon l'une quelconque des revendications 1 à 5, caractérisé en ce que des anticorps marqués anti-virus HTLV-III sont utilisés dans ledit procédé.

**10.** Procédé selon les revendications 8 ou 9, caractérisé en ce que le virus HTLV-III, les fractions recombinantes du virus HTLV-III, ou les antigènes du virus HTLV-III présents dans l'échantillon sont détectés par ledit procédé.

**11.** Procédé selon l'une quelconque des revendications 1 à 7, caractérise en ce que le protocole de discrimination est choisi parmi la technique ELISA et les techniques d'essai par immuno-précipitation et immunofluorescence.

**12.** Procédé selon l'une quelconque des revendications 1 à 5, caractérisé en ce qu'on fait réagir un anticorps anti-virus HTLV-III avec un antigène du virus HTLV-III, pour former un complexe immun, et on fait réagir ce dernier avec un anticorps secondaire marqué pour conduire à un produit aisément dosable.

**13.** Procédé selon la revendication 12, caractérisé en ce que l'anticorps secondaire est marqué avec un enzyme qui peut libérer un produit coloré.

**14.** Procédé selon la revendication 12, caractérisé en ce que l'anticorps secondaire est marqué à l' $^{125}$I radioactif.

**15.** Procédé selon l'une quelconque des revendications 1 à 5, caractérisé en ce que le protocole de discrimination est celui du type Western Blot, selon lequel les bandes de protéines du virus HTLV-III sont obtenues par électrophorèse du virus HTLV-III sur gel de polyacrylamide en présence de dodécylsulfate de sodium, les bandes de protéines dans le gel sont transférées sur une plaque de nitrocellulose, la plaque est saturée en une protéine sans relation avec les précédentes, la plaque est découpée en bandelettes donnant le profil des antigènes du virus HTLV-III, et les antigènes sont utilisés pour détecter les anticorps dans ledit échantillon.

**16.** Procédé selon l'une quelconque des revendications 1 à 5, caractérisé en ce que ledit antigène est obtenu en concentrant le virus HTLV-III par ultracentrifugation des surnageants de la culture de virus, en éliminant les lipides par centrifugation dans une solution de saccharose à 20 % (P/P) dans un tampon TNE, pour créer un gradient de saccharose, en fractionnant le gradient de saccharose et en isolant les bandes d'antigènes par analyse, sur des parties aliquotes de chaque fraction, de l'activité transcriptase inverse spécifique du virus HTLV-III.

**17.** Procédé selon les revendications 8 ou 9, caractérisé en ce que ledit échantillon comprend une culture de tissu ou des cellules primaires de liquides biologiques.

**18.** Procédé selon la revendication 2, caractérisé en ce que l'antigène p41 de l'enveloppe est marqué.

**19.** Procédé selon la revendication 2, caractérisé en ce que l'antigène p24 de nucléocapside est marqué.

**20.** Ensemble d'analyse spécifique du SIDA comprenant une enceinte compartimentée, caractérisé en ce qu'un compartiment de l'enceinte au moins contient au moins un rétrovirus lymphotropique, désigné sous le nom de virus HTLV-III, qui est normalement cytopathogène à l'égard de sa cible préférentielle, à savoir les cellules $T_4$ inductrices, qui a une densité de 1,16 g/ml déterminée par gradient de densité de saccharose, qui a un antigène principal p24 de nucléocapside, ayant un poids moléculaire de 24 000 et un antigène principal p41 d'enveloppe, ayant un poids moléculaire de 41 000 et qui présente une réactivité croisée limitée avec tous les autres sous-groupes de HTLV connus, des fractions recombinantes du virus HTLV-III, des anticorps anti-virus HTLV-III ou des antigènes du virus HTLV-III, caractérise en ce qu'un compartiment de l'enceinte au moins contient au moins une protéine HTLV-III obtenue à partir d'une culture de cellules immortelles ou à partir d'un anticorps anti-protéines HTLV-III, ladite protéine HTLV-III excluant les protéines révélées dans EP-A-138,667.

**21.** Coffret d'analyse spécifique du SIDA selon la revendication 20, caractérisé en ce qu'un compartiment de ladite enceinte au moins contient une protéine HTLV-III choisie parmi p24, p32, p39, p41, p48, p55, p60, p65 et p130.

**22.** Ensemble d'analyse selon la revendication 21, caractérisé en ce que la protéine HTLV-III est la p24.

**23.** Ensemble d'analyse selon la revendication 21, caractérisé en ce que la protéine HTLV-III est la p41.

**24.** Ensemble d'analyse selon la revendication 21, caractérisé en ce que la protéine HTLV-III est la p130.

**25.** Ensemble d'analyse selon l'une quelconque des revendications 20 à 24, caractérisé en ce que ledit compartiment contient des anticorps anti-virus HTLV-III.

**26.** Ensemble d'analyse selon l'une quelconque des revendications 20 à 24, caractérisé en ce que ledit compartiment contient le virus HTLV-III.

**27.** Ensemble d'analyse selon l'une quelconque des revendications 20 à 24, caractérisé en ce que ledit compartiment contient des antigènes du virus HTLV-III.

**28.** Ensemble d'analyse selon l'une quelconque des revendications 20 à 24, caractérisé en ce que ledit compartiment contient un lysat du virus HTLV-III.

**29.** Ensemble d'analyse selon la revendication 28, caractérisé en ce qu'il comprend :
a) des plaques de microtitration recouvertes d'un lysat du virus HTLV-III et traitées par des solutions protéiniques pour empêcher toute adsorption ultérieure des protéines sur les plaques par d'autres voies qu'une interaction antigène-anticorps ;
b) un sérum humain comme témoin positif pour une réactivité donnée et un titre donné d'anticorps anti-antigènes du virus HTLV-III ;
c) un sérum comme témoin négatif sans titre d'anticorps anti-antigènes du virus HTLV-III ;
d) un anticorps secondaire marqué par un enzyme, anti-immunoglobulines humaines et une solution diluante pour cet anticorps secondaire consistant en un sérum témoin de l'espèce animale de laquelle provient l'anticorps secondaire ;
e) un mélange de substrat orthophénylènediamine et $H_2O_2$ ;
f) une solution pour stopper la réaction enzymatique ;
g) un tampon de lavage ; et
h) une solution saline de phosphate tamponnée.

**30.** Ensemble d'analyse selon la revendication 26, caractérisé en ce que l'enceinte comporte une pluralité de puits, des plaques qui sont recouvertes préalablement de HTLV-III et des réactifs ELISA pour la détection enzymatique consistant en un sérum témoin de chèvre et une peroxydase, une IgG de chèvre antihumaine marquée et un indicateur de changement de couleur.

**31.** Ensemble d'analyse selon la revendication 30, caractérisé en ce que l'indicateur de changement de couleur consiste en l'orthophénylènediamine et le peroxyde d'hydrogène dans une solution tampon de phosphate et citrate.

**32.** Ensemble d'analyse selon la revendication 31, caractérisé en ce que le HTLV-III se trouve sous forme d'un lysat.

**33.** Ensemble d'analyse selon la revendication 26, caractérisé en ce que l'enceinte contient en outre une solution saline de phosphate tamponnée et une IgG antisérum de chèvre couplée à la fluorescine.

**34.** Ensemble d'analyse selon la revendication 26, caractérisé en ce que l'enceinte contient en outre une plaque de nitrocellulose et un gel polyacrylamide de surface en présence de dodécylsulfate de sodium, des tensio-actifs, des modificateurs de pH, de l'albumine de sérum bovin, un fragment Fab humain et une réserve de sérum étalon dilué de chèvre et une immunoglobuline antihumaine de chèvre marquée à $I^{125}I$.

**35.** Ensemble d'analyse selon la revendication 27, caractérisé en ce que l'enceinte contient :
a) des plaques de nitrocellulose avec des bandes de protéines HTLV-III déjà séparées par électrophorèse sur un gel et convenablement immobilisées avec des solutions protéiques pour empêcher toute liaison protéique ultérieure non spécifique ;
b) un sérum humain comme témoin positif ;
c) un sérum humain comme témoin négatif ;
d) un anticorps secondaire anti-immunoglobulines humaines provenant d'un animal-hôte approprié et marqué par un indicateur radioactif ;
e) une solution diluante ;
f) une solution de lavage ;

19

EP 0 181 374 B1

g) des tubes à essai avec bouchons.

36. Ensemble d'analyse selon la revendication 35, caractérisé en ce que la solution de lavage contient du désoxycholate de sodium à 0,5 %, du NaCl 0,1M, du Triton® X-100 à 0,5 %, du fluorure de phénylméthylsulfonyle 0,1mM et du phosphate de sodium 10mM ; pH 7,5.

37. Ensemble d'analyse selon la revendication 20, caractérisé en ce qu'un compartiment au moins de l'enceinte contient l'antigène de l'enveloppe p41.

38. Ensemble d'analyse selon la revendication 20, caractérisé en ce qu'un compartiment au moins de l'enceinte contient l'antigène de nucléocapside p24.

39. Ensemble d'analyse selon la revendication 20, caractérisé en ce que l'enceinte contient :
a) des plaques de nitrocellulose avec des bandes de protéines HTLV-III déjà séparées par électrophorèse sur gel et convenablement immobilisées avec des solutions protéiques pour empêcher toute liaison protéique ultérieure non spécifique ;
b) un sérum humain comme témoin positif ;
c) un sérum humain comme témoin négatif ;
d) un anticorps secondaire anti-immunoglobuline humaine provenant d'un animal-hôte approprié et marqué par un indicateur radioactif ;
e) une solution diluante ;
f) une solution de lavage ;
g) des tubes à essai avec bouchons.

40. Ensemble d'analyse selon la revendication 39, caractérisé en ce que la solution de lavage contient du désoxycholate de sodium à 0,5 %, du NaCl 0,1M, du Triton® X-100 à 0,5 %, du fluorure de phénylméthylsulfonyle 0,1mM et du phosphate de sodium 10mM ; pH 7,5.

41. Ensemble d'analyse selon la revendication 39, caractérisé en ce que l'enceinte comprend en outre des anticorps primaires soit anti-antigènes p41 de l'enveloppe, soit anti-antigènes p24 de nucléocapside, des anticorps secondaires anti-anticorps primaires, des solutions tampons pH et des tensioactifs.

42. Ensemble d'analyse selon la revendication 37, caractérisé en ce que l'enceinte contient un récipient réactionnel pour additionner la matière à analyser dans laquelle les antigènes p41 de l'enveloppe ou les antigènes p24 de nucléocapside sont recherchés.

**Patentansprüche**

1. Verfahren zum Nachweis eines lymphotropen Retrovirus, der als HTLV-III-Virus bezeichnet wird, normalerweise gegen sein bevorzugtes Angriffsziel, Helfer-T4-Zellen, zytopathogen ist, nach der Sucrose-Dichtebandbestimmung eine Dichte von 1,16 g/ml hat, ein größeres Kernantigen p24 mit dem Molekulargewicht 24.000 und ein größeres Hüllantigen p41 mit dem Molekulargewicht 41.000 aufweist und nur begrenzte Kreuzreaktivität mit allen anderen bekannten HTLV-Untergruppen, rekombinanten Fraktionen des HTLV-III-Virus, Antikörpern zum HTLV-III-Virus oder Antigene des HTLV-III-Virus in einer Probe zeigt, dadurch gekennzeichnet, daß die Probe einem kompetitiven Immunbestimmungs-Diskriminierungsverfahren und/oder einem Western-Blot-Bestimmungsverfahren unterzogen wird, wobei das Verfahren HTLV-III-Antigene verwendet, die aus einer immortalisierten Zellkultur oder von unabhängig abgeleiteten Antikörpern zu HTLV-III gewonnen sind, wobei das Verfahren für HTLV-III-Proteine ausgenommen Antigene nach der europäischen Patentanmeldung Nr. 138 667 oder für Antikörper hierzu spezifisch ist.

2. Verfahren zum Nachweis eines lymphotropen Retrovirus nach Anspruch 1, dadurch gekennzeichnet, daß die Probe einem kompetitiven Immunbestimmungs-Diskriminierungsverfahren und/oder einem Western-Riot-Bestimmungsverfahren unterzogen wird, das für ein aus p24, p32, p39, p41, p48, p55, p60, p65 und p130 ausgewähltes HTLV-III-Protein spezifisch ist.

3. Verfahren nach Anspruch 2, dadurch gekennzeichnet, daß das HTLV-III-Protein p24 ist.

20

4. Verfahren nach Anspruch 2, dadurch gekennzeichnet, daß das HTLV-III-Protein p41 ist.

5. Verfahren nach Anspruch 2, dadurch gekennzeichnet, daß das HTLV-III-Protein p130 ist.

6. Verfahren nach Anspruch 1, dadurch gekennzeichnet, daß das HTLV-III-Virus zum Nachweis von Antikörpern zum HTLV-III-Virus in der Probe verwendet wird.

7. Verfahren nach einem der Ansprüche 1 bis 5, dadurch gekennzeichnet, daß ein Antigen des HTLV-III-Virus zum Nachweis von Antikörpern zum HTLV-III-Virus in der Probe verwendet wird.

8. Verfahren nach einem der Ansprüche 1 bis 5, dadurch gekennzeichnet, daß das markierte HTLV-III-Virus, markierte rekombinante Fraktionen des HTLV-III-Virus, oder markierte Antigene des HTLV-III-Virus in dem Verfahren verwendet werden.

9. Verfahren nach einem der Ansprüche 1 bis 5, dadurch gekennzeichnet, daß markierte Antikörper zum HTLV-III-Virus in dem Verfahren verwendet werden.

10. Verfahren nach Anspruch 8 oder 9, dadurch gekennzeichnet, daß das HTLV-III-Virus, rekombinante Fraktionen des HTLV-III-Virus oder Antigene des HTLV-III-Virus in der Probe durch das Verfahren nachgewiesen werden.

11. Verfahren nach einem der Ansprüche 1 bis 7, dadurch gekennzeichnet, daß das Diskriminierungsverfahren aus ELISA, Immunausfällungs- und Immunfluoreszenz-Bestimmungsverfahren ausgewählt ist.

12. Verfahren nach einem der Ansprüche 1 bis 5, dadurch gekennzeichnet, daß ein Antikörper zum HTLV-III-Virus mit einem Antigen des HTLV-III-Virus zur Bildung eines Immunkomplexes reagiert wird, und daß der Immunkomplex mit einem markierten sekundären Antikörper zur Bildung eines leicht quantifizierbaren Produkts reagiert wird.

13. Verfahren nach Anspruch 12, dadurch gekennzeichnet, daß der sekundäre Antikörper mit einem Enzym etikettiert wird, das ein gefärbtes Produkt erzeugen kann.

14. Verfahren nach Anspruch 12, dadurch gekennzeichnet, daß der sekundäre Antikörper mit radioaktiven $^{125}$I etikettiert wird.

15. Verfahren nach einem der Ansprüche 1 bis 5, dadurch gekennzeichnet, daß das Diskriminierungsverfahren dem Western-Blot-Typ angehört, bei welchem Proteinbänder des HTLV-III-Virus durch Elektrophorese des HTLV-III-Virus auf einem Polyacrylamid-Gel in Anwesenheit von Natrium-Dodecylsulfat gebildet werden, die Proteinbänder in dem Gel auf ein Nitrocelluloseblatt übertragen werden, das Blatt mit einem nicht verwandten Protein gesättigt wird, das Blatt dann in Streifen aufgetrennt wird, welche das Profil das Antigens zum HTLV-III-Virus darstellen, und die Antigene zum Nachweis von Antikörpern in der Probe verwendet wird.

16. Verfahren nach einem der Ansprüche 1 bis 5, dadurch gekennzeichnet, daß das Antigen durch Konzentrieren des HTLV-III-Virus durch Ultrazentrifugieren aus Viruskultur-Deckflüssigkeiten, Entfernen von Lipiden durch Zentrifugieren durch (nach Gewicht) 20 prozentige Sucrose in TNE-Puffer zur Bildung eines Sucrose-Gradienten, Unterteilen des Sucrose-Gradienten in Fraktionen, und Isolieren von Antigen-Bändern durch Untersuchen von Aliquoten jeder Fraktion auf HTLV-III-Virus-spezifische Reverse-Transcriptase-Aktivität gewonnen wird.

17. Verfahren nach Anspruch 8 oder 9, dadurch gekennzeichnet, daß die Probe Gewebekulturmaterial oder Primärzellen von Körperflüssigkeiten enthält.

18. Verfahren nach Anspruch 2, dadurch gekennzeichnet, daß das Hüllantigen p41 markiert wird.

19. Verfahren nach Anspruch 2, dadurch gekennzeichnet, daß das Kernantigen p24 markiert wird.

20. AIDS-spezifische Testausrüstung mit einem in Kammern unterteilten Behältnis, dadurch gekennzeichnet, daß mindestens eine Kammer des Behältnisses mindestens ein lymphotropes Retrovirus enthält, das als HTLV-III-Virus bezeichnet wird und normalerweise gegen sein bevorzugtes Angriffsziel, Helfer-T4-Zellen, zytopathogen ist, nach der Sucrose-Dichtebandbestimmung eine Dichte von 1,16 g/ml hat, ein größeres Kernantigen p24 mit dem Molekulargewicht 24.000 und eine größeres Hüllantigen p41 mit dem Mulekulargewicht 41.000 aufweist und nur begrenzte Kreuzreaktivität mit allen anderen bekannten HTLV-Untergruppen, rekombinanten Fraktionen des HTLV-III-Virus, Antikörpern zum HTLV-III-Virus und Antigenen des HTLV-III-Virus zeigt;

dadurch gekennzeichnet, daß mindestens eine Kammer des Behältnisses mindestens ein HTLV-III-Protein, ausgenommen Proteine nach EP-A-138 667, enthält, die aus einer immortalisierten Zellkultur oder aus einem unabhängig abgeleiteten Antikörper zu HTLV-III-Proteinen gewonnen ist.

21. AIDS-spezifische Testausrüstung nach Anspruch 20, dadurch gekennzeichnet, daß mindestens eine Kammer des Behältnisses ein HTLV-III-Protein enthält, das aus p24, p32, p39, p41, p48, p55, p60, p65 und p130 ausgewählt ist.

22. Testausrüstung nach Anspruch 21, dadurch gekennzeichnet, daß das HTLV-III-Protein p24 ist.

23. Testausrüstung nach Anspruch 21, dadurch gekennzeichnet, daß das HTLV-III-Protein p41 ist.

24. Testausrüstung nach Anspruch 21, dadurch gekennzeichnet, daß das HTLV-III-Protein p130 ist.

25. Testausrüstung nach einem der Ansprüche 20 bis 24, dadurch gekennzeichnet, daß die mindestens eine Kammer Antikörper zum HTLV-III-Virus enthält.

26. Testausrüstung nach einem der Ansprüche 20 bis 24, dadurch gekennzeichnet, daß die mindestens eine Kammer das HTLV-III-Virus enthält.

27. Testausrüstung nach einem der Ansprüche 20 bis 24, dadurch gekennzeichnet, daß die mindestens eine Kammer Antigene des HTLV-III-Virus enthält.

28. Testausrüstung nach einem der Ansprüche 20 bis 24, dadurch gekennzeichnet, daß die mindestens eine Kammer HTLV-III-Viruslysat enthält.

29. Testausrüstung nach Anspruch 28, dadurch gekennzeichnet, daß sie aufweist,
a) Mikrotiter-Plättchen, die mit HTLV-III-Viruslysat überzogen und mit Proteinlösungen behandelt sind, um weitere Adsorption von Proteinen an den Plättchen durch andere als Antigen-Antikörper-Interaktion zu verhindern,
b) positives menschliches Kontrollserum bekannter Reaktivität und bekanntem Antikörpertiter zu Antigenen des HTLV-III-Virus,
c) negatives Kontrollserum ohne Antikörpertiter zu Antigenen des HTLV-III-Virus,
d) einen Enzym-markierten sekundären Antikörper, der gegen menschliche Immunglobuline reaktiv ist, und eine Verbindungslösung für den sekundären Antikörper, bestehend aus Normalserum der Tierart, in welcher der sekundäre Antikörper erzeugt ist,
e) ein Substratgemisch aus Orthophenylendiamin und $H_2O_2$,
f) eine Lösung zum Stoppen der Enzymreaktion,
g) einen Waschpuffer, und
h) phosphatgepufferte Salzlösung

30. Testausrüstung nach Anspruch 26, dadurch gekennzeichnet, daß das Behältnis eine Vielzahl von Behältern, weiter/Plättchen, die vor Gebrauch mit HTLV-III überzogen werden, und ELISA-Materialien zum Enzymnachweis enthält, bestehend aus normalem Ziegenserum und Peroxidase, markiertem Ziegen-Antihuman IgG, und einem Farbwechsel-Indikator.

31. Testausrüstung nach Anspruch 30, dadurch gekennzeichnet, daß der Farbwechsel-Indikator aus Orthophenylendiamin und Wasserstoffperoxid in Phosphatzitrat-Puffer besteht.

32. Testausrüstung nach Anspruch 31, dadurch gekennzeichnet, daß das HTLV-III in Form eines Lysats

EP 0 181 374 B1

vorhanden ist.

33. Testausrüstung nach Anspruch 26, dadurch gekennzeichnet, daß das Behältnis weiter eine phosphatgepufferte Salzlösung und Fluoreszein-konjugiertes Ziegen-Antiserum IgG enthält.

34. Testausrüstung nach Anspruch 26, dadurch gekennzeichnet, daß das Behältnis außerdem ein Nitrozelluloseblatt und ein Polyacrylamid-Plattengel in Anwesenheit von Natrium-Dodecylsulfat, oberflächenaktive Substanzen, pH-Modifizierer, Rinderserum-Albumin, menschliches Fab, und einen Vorrat von verdünntem normalem Ziegenserum und $^{125}$I-markiertes Ziegen-Antihuman-Immunglobulin enthält.

35. Testausrüstung nach Anspruch 27, dadurch gekennzeichnet, daß das Behältnis enthält
a) Nitrozellulose-Blätter mit HTLV-III-Proteinbändern, die bereits durch Gel-Elektrophorese aufgespalten und in geeigneter Weise mit Proteinlösungen blockiert sind, um eine weitere nichtspezifische Proteinbindung zu unterbinden,
b) ein positives menschliches Kontrollserum,
c) ein negatives menschliches Kontrollserum,
d) einen sekundären Antikörper zu menschlichen Immunglobulin, das in einem geeigneten tierischen Wirt erzeugt und mit einer radioaktiven Markierung etikettiert wurde,
e) Verdünnungslösung
f) Waschlösung,
g) Teströhrchen mit Kappen

36. Testausrüstung nach Anspruch 35, dadurch gekennzeichnet, daß die Waschlösung 0,5 % Natrium-Deoxicholat, 0,1 M NaCl, 0,5 % Triton X-100, 0,1 mM Phenylmethylsulfonyl-Fluorid und 10 mM Natriumphosphat, pH-Wert 7,5, enthält.

37. Testausrüstung nach Anspruch 20, dadurch gekennzeichnet, daß mindestens eine Kammer des Behältnisses das Hüllantigen p$^{41}$ enthält.

38. Testausrüstung nach Anspruch 21, dadurch gekennzeichnet, daß mindestens eine Kammer des Behältnisses das Kernantigen p$^{24}$ enthält.

39. Testausrüstung nach Anspruch 20, dadurch gekennzeichnet, daß das Behältnis enthält
a) Nitrozellulose-Blätter mit HTLV-III-Proteinbändern, die bereits durch Gel-Elektrophorese aufgespalten und in geeigneter Weise mit Proteinlösungen blockiert sind, um eine weitere nichtspezifische Proteinbindung zu unterbinden,
b) ein positives menschliches Kontrollserum,
c) ein negatives menschliches Kontrollserum,
d) einen sekündären Antikörper zu menschlichen Immunglobulin, das in einem geeigneten tierischen Wirt erzeugt und mit einer radioaktiven Markierung etikettiert wurde,
e) Verdünnungslösung,
f) Waschlösung
g) Teströhrchen mit Kappen

40. Testausrüstung nach Anspruch 39, dadurch gekennzeichnet, daß die Waschlösung 0,5 % Natrium Deoxicholat, 0,1 M NaCl, 0,5 % Triton X-100, 0,1 mM Phenylmethylsulfonyl-Fluorid und 10 mM Natriumphosphat, pH-Wert 7,5, enthält.

41. Testausrüstung nach Anspruch 39, dadurch gekennzeichnet, daß das Behältnis außerdem primäre Antikörper, die entweder mit den Hüllantigenen p$^{41}$ oder mit dem Kernantigen p24 reaktiv sind, sekundäre Antikörper gegen die primären Antikörper, pH-Puffer und oberflächenaktive Substanzen enthält.

42. Testausrüstung nach Anspruch 37, dadurch gekennzeichnet, daß das Behältnis ein Reaktionsgefäß zur Zugabe von Testmaterial enthält, das auf Hüllantigene p41 oder das Kernantigen p24 auszuwerten ist.

23

FIG.1

FIG.2

FIG.3

FIG.4

FIG.5